# EUROPEAN PATENT APPLICATION

(11) **EP 1 967 162 A2**
(43) Date of publication of application: **10.09.2008**
(21) Application number: 08102153.7
(22) Date of filing: 29.02.2008
(51) Int. Cl.: A61F 2/16

(54) **Lens delivery system cartridge**

(30) Priority: 09.03.2007 US 684337
(71) Applicant: ALCON RESEARCH, LTD., 76134-2099 TX, Texas Fort Worth (US)
(72) Inventor: DOWNER, David A., Fort Worth, TX 76137 (US)
(74) Representative: Hanna, Peter William Derek

(57) **Abstract**

A cartridge for an intraocular lens (IOL) delivery system is provided with two body halves that fold open along a longitudinal axis about two pairs of hinges (18,20). The interior of the cartridge is hollow and contains a folding chamber (22) communicating with a nozzle (14). An IOL is placed in the folding chamber which has a pair of rails (28), each rail projecting from a body half and into the hollow interior folding chamber, that help to assist in providing an initial fold of the IOL and help prevent rotation of the IOL during injection.

## Description

This invention relates to intraocular lenses (IOLs) and more particularly to cartridges for use with devices use to inject IOLs into an eye.

### Background of the Invention

The human eye in its simplest terms functions to provide vision by transmitting and refracting light through a clear outer portion called the cornea, and further focusing the image by way of the lens onto the retina at the back of the eye. The quality of the focused image depends on many factors including the size, shape and length of the eye, and the shape and transparency of the cornea and lens.

When trauma, age or disease cause the lens to become less transparent, vision deteriorates because of the diminished light which can be transmitted to the retina. This deficiency in the lens of the eye is medically known as a cataract. The treatment for this condition is surgical removal of the lens and implantation of an artificial lens or IOL.

While early IOLs were made from hard plastic, such as polymethylmethacrylate (PMMA), soft, foldable IOLs made from silicone, soft acrylics and hydrogels have become increasingly popular because of the ability to fold or roll these soft lenses and insert them through a smaller incision. Several methods of rolling or folding the lenses are used. One popular method is an injector cartridge that folds the lenses and provides a relatively small diameter lumen through which the lens may be pushed into the eye, usually by a soft tip plunger. The most commonly used injector cartridge design is illustrated in U.S. Patent No. 4,681,102 (Bartell), and includes a split, longitudinally hinged cartridge. Similar designs are illustrated in U.S. Patent Nos. 5,494,484 and 5,499,987 (Feingold) and 5,616,148 and 5,620,450 (Eagles, et al.). fu an attempt to avoid the claims of U.S. Patent No. 4,681,102, several solid cartridges have been investigated, see for example U.S. Patent No. 5,275,604 (Rheinish, et al.) and 5,653,715 (Reich, et al.).

These prior art devices were intended to inject an IOL into the posterior chamber of an aphakic eye following removal of a cataractous lens. In such an eye, there is additional room to maneuver the IOL, such as turning the IOL over if the IOL is injected upside down. As a result, these prior art cartridges did not contain features to prevent the IOL from rotating within the cartridge.

With respect to IOLs intended to be planted into a phakic eye (an eye in which the natural lens remains), these lenses are either placed in the anterior chamber, or placed in the posterior chamber between the natural lens and the iris. Both styles of phakic lenses have a curvature or vault, requiring that the IOL be placed right side up. And in either case, the amount of room to maneuver the IOL is greatly restricted. Therefore, it is extremely important that the IOL be injected into the eye in the correct orientation.

Accordingly, a need continues to exist for an intraocular lens injection cartridge that helps prevent the lens from rotating during injection.

### Brief Summary of the Invention

The present invention improves upon prior art by providing a cartridge for an IOL delivery system that folds open along its longitudinal axis on two pairs of hinges, and/or which has a pair of rails, in accordance with claims which follow. The interior of the cartridge is hollow and contains a folding chamber connected to a nozzle. The folding chamber has a pair of rails that help to assist in providing an initial fold of the IOL and help prevent rotation of the IOL during injection.

It is accordingly an objective of the present invention to provide a cartridge for a lens delivery system that helps to prevent rotation of the lens during insertion.

It is a further objective of the present invention to provide a cartridge for a lens delivery system that has a folding chamber with a pair of rails.

It is a further an objective of the present invention to provide a cartridge for a lens delivery system that folds open along its longitudinal axis on two pairs of hinges.

Other objectives, features and advantages of the present invention will become apparent with reference to the drawings, and the following description of the drawings and claims.

### Brief Description of the Drawings

FIG. 1 is an enlarged top perspective view of the lens delivery system cartridge of the present invention.
FIG. 2 is an enlarged bottom perspective view of the lens delivery system cartridge of the present invention.
FIG. 3 is an enlarged side elevational view of the lens delivery system cartridge of the present invention.
FIG. 4 is an enlarged proximal end view of the lens delivery system cartridge of the present invention shown in the closed position and containing a folding IOL.

### Detailed Description of the Preferred Embodiments

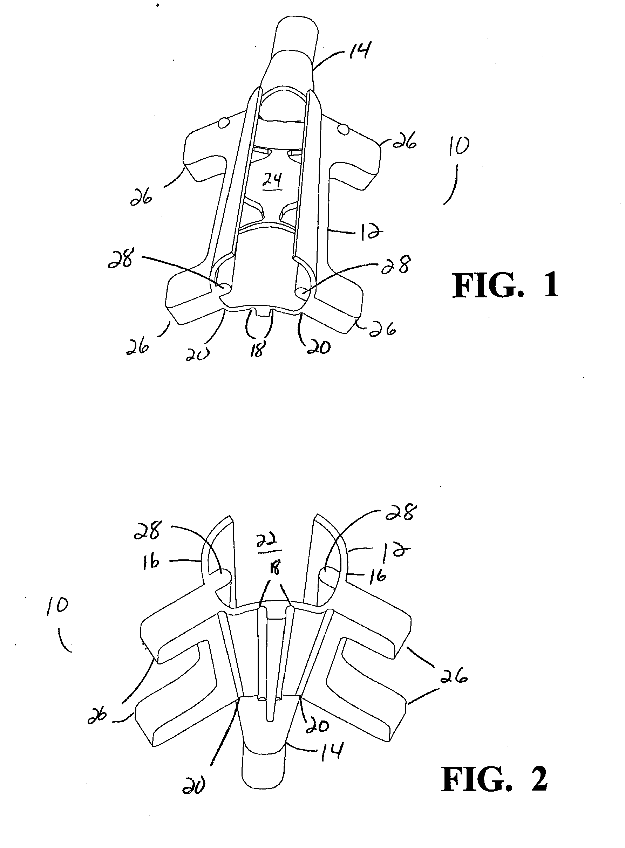

As best seen in FIGS. 1-3, lens cartridge 10 of the present invention generally includes body 12 and nozzle 14. Cartridge 10 can be molded from any suitable thermoplastic, such as polypropylene, and the thermoplastic may contain a lubricity enhancing agent such as those disclosed in U.S. Pat. No.5,716,364. Nozzle 14 may be integrally formed with body 12. Nozzle 14 preferably is tubular, round, oval or elliptical in cross-section and has a cross-sectional area of between around 1.0 mm² to around 2.6 mm². Body 12 is formed by two hemispherical halves 16 connected by hinge pairs 18 and 20. When collapsed or folded together, halves 16 form body 12 having a hollow interior folding chamber 22 into which IOL 24 may be placed. This folding may be assisted by use of finger grips 26. Projecting into interior 22 of body 12 is a pair of rails 28.

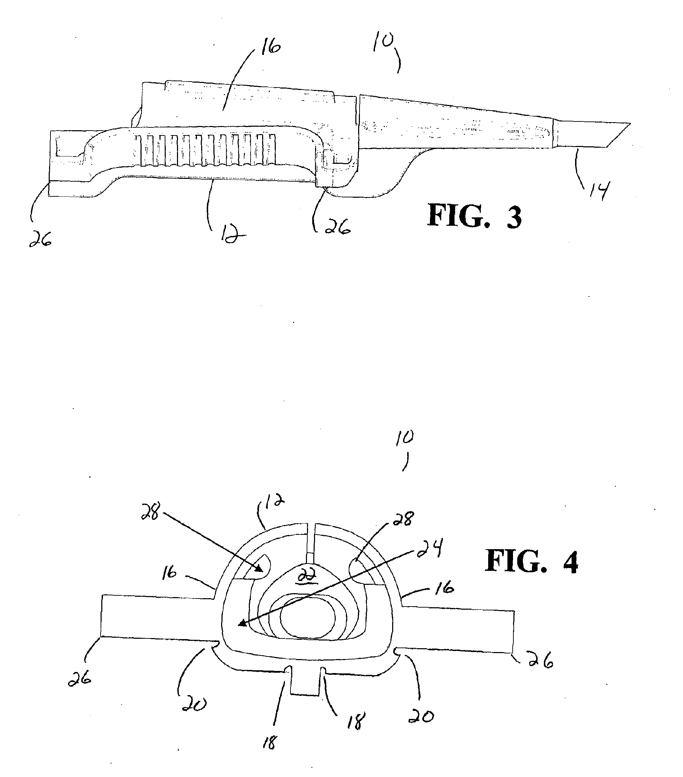

In use, IOL 24 is placed within chamber 22 of body 12 below rails 28. Halves 16 are collapsed together, initially by flexing of hinge pairs 18, which impart an initial fold on IOL 24 that ensures that IOL 24 bends or flexes downwardly toward hinge pair 18. Rails 28 prevent IOL 24 from rotating within body 12 during folding. As halves 16 continue to be collapsed together, further folding of IOL 24 is accomplished by flexing of hinge pairs 20, as best seen in FIG. 4.

While certain embodiments of the present invention have been described above, these descriptions are given for purposes of illustration and explanation. Variations, changes, modifications and departures from the systems and methods disclosed above may be adopted without departure from the scope of the present invention.

## Claims

1. An intraocular lens delivery system cartridge (10), comprising:
(a) a body (12), the body formed by a pair of body halves (16) connected by a longitudinal hinge arrangement and defining a hollow interior folding chamber (22) in the body adapted to receive an intraocular lens (24); and
(b) a tubular nozzle (14) communicating with the body through which a folded lens may be injected,
**characterized in that** the hinge arrangement is defined by two pairs of hinges (18,20).

2. The intraocular lens delivery system cartridge of claim 1, wherein a first pair of hinges (18) is arranged one hinge to each side of a longitudinal axis of the body, and a second pair of hinges (20) is spaced laterally from said first pair of hinges.

3. The intraocular lens delivery system cartridge of claim 1 or claim 2, wherein the two pairs of hinges (18,20) are flexible hinges, such that collapsing the body halves (16) together about the first pair of hinges (18) by flexing the hinges imparts an initial fold on an intraocular lens (24) placed within the chamber (22), and further folding of the intraocular lens is accomplished by flexing the second pair of hinges (20).

4. The intraocular lens delivery system cartridge of any of claims 1 to 3, further comprising a pair of rails (28), each rail projecting from a body half and into the hollow interior folding chamber, adapted to prevent an intraocular lens (24) placed in the chamber (22) from rotating within the body during folding.

5. The intraocular lens delivery system cartridge of claim 4, wherein the pair of rails (28) is arranged so as to ensure an intraocular lens (24) placed in the chamber (22) is caused to bend or flex downwardly.

6. An intraocular lens delivery system cartridge (10), comprising:
(a) a body (12), the body formed by a pair of body halves (16) connected by a longitudinal hinge arrangement and defining a hollow interior folding chamber (22) in the body adapted to receive an intraocular lens (24); and
(b) a tubular nozzle (14) communicating with the body through which a folded lens may be injected,
**characterized by** a pair of rails (28), each rail projecting from a body half and into the hollow interior folding chamber, adapted to prevent an intraocular lens (24) placed in the chamber (22) from rotating within the body during folding.

7. The intraocular lens delivery system cartridge of claim 6, wherein the pair of rails (28) is arranged so as to ensure an intraocular lens (24) placed in the chamber (22) is caused to bend or flex downwardly.

8. The intraocular lens delivery system cartridge of claim 6 or claim 7, wherein the hinge arrangement is defined by two pairs of hinges (18,20).

9. The intraocular lens delivery system cartridge of any of claims 6 to 8, wherein a first pair of hinges (18) is arranged one hinge to each side of a longitudinal axis of the body, and a second pair of hinges (20) is spaced laterally from said first pair of hinges.

10. The intraocular lens delivery system cartridge of claim 9, wherein the two pairs of hinges (18,20) are flexible hinges, such that collapsing the body halves (16) together about the first pair of hinges (18) by flexing the hinges imparts an initial fold on an intraocular lens (24) placed within the chamber (22), and further folding of the intraocular lens is accomplished by flexing the second pair of hinges (20).
